# EUROPEAN PATENT SPECIFICATION

(11) **EP 0 334 576 B1**
(45) Date of publication and mention of the grant of the patent: **06.10.1993**
(21) Application number: 89302725.0
(22) Date of filing: 20.03.1989
(51) Int. Cl.: A61K 45/06, A61K 31/71, A61K 31/505, A61K 33/24, A61K 31/40

(54) **Intraperitoneal treatment by chemotherapeutic agent and non-steroidal antiinflammatory drug**
Intraperitonealbehandlung mit einem chemotherapeutischen Mittel und einem nichtsteroiden antiinflammatorischen Arzneistoff
Traitement intrapéritonéal par agent chimothérapeutique et médicament anti-inflammatoire non stéroidien

(30) Priority: 21.03.1988 US 171059
(43) Date of publication of application: 27.09.1989
(73) Proprietor: ETHICON INC., Somerville New Jersey 08876 (US)
(72) Inventor: Sheffield, Warren D., Lebanon, NJ 08833 (US); diZerega, Gere S., Pasadena, CA 91107 (US)
(74) Representative: Fisher, Adrian John

(56) References cited:
- GB-A- 955 086
- US-A- 4 720 504
- OBSTETRICAL AND GYNOCOLOGICAL SURVEY, vol. 42, 1987 Williams & Wilkins M. MARKMAN "Intraperitoneal Chemotherapy as Treatment of Ovarian Carcinoma: Why, How and When?" pages 533-539
- O. ZEKERT "Austria Codex Fachinformation 1987/88" Oesterreichische Apotheker-Verlagsgesellschaft m.b.H. Wien, 1987

## Description

This application is a continuation-in-part of our copending application Serial No. 171,059, filed March 21, 1988.

The invention relates to an intraperitoneal treatment by a novel combination of an antineoplastic chemotherapeutic agent and a non-steroidal anti-inflammatory drug ("NSAID").

Intraperitoneal chemotherapeutic treatment of ovarian and other types of cancer that occur in the peritoneal cavity is an experimental procedure that is being investigated. A recent review of this type of procedure applied to the treatment of ovarian cancer is found in Markman, "Intraperitoneal Chemotherapy as Treatment of Ovarian Carcinoma: Why, How, and When?", Obstetrical and Gynecological Survey, Vol. 42, No. 9, pages 533-538 (1987). Other articles relating to this procedure are Myers, "The Clinical Setting and Pharmacology of Intraperitoneal Chemotherapy: An Overview", Seminars in Oncology, Vol XII, No. 2, Suppl 4 (September), 1985, pages 12-16; Speyer et al., "Phase I and Pharmacological Studies of 5-Fluorouracil Administered Intraperitoneally", Cancer Research 40, 567-572, March 1980; and Howell et al., "Intraperitoneal Cisplatin with Systemic Thiosulfate Protection", Annals of Internal Medicine, 1982;97:845-851. There are several different ways of carrying out the procedure. In one variation, a large volume (up to about two liters) of liquid containing the antineoplastic chemotherapeutic agent ("CAA") is administered intraperitoneally through a catheter. At the end of the treatment, perhaps two or three hours later, the liquid will be withdrawn through the catheter. In another variation, liquid containing the CAA is administered intraperitoneally substantially continually over a period of time (for perhaps two to four weeks).

Chemotherapeutic antineoplastic agents are powerful chemicals; they all have a significant potential for causing irritation of tissue. It has been found that when they are administered intraperitoneally by the procedures outlined above, fibrin-like adhesions may be formed in response to the irritation, which adhesions interfere with the circulation of the liquid in the peritoneal cavity or with the flow of liquid through the catheter, either into the peritoneal cavity or out of it when the liquid is withdrawn. In this respect, see Pfeifle et al., "Totally Implantable System for Peritoneal Access", Journal of Clinical Oncology, Vol. 2, No. 11, pages 1277-1280 (November, 1984). The adhesion formation may be severe enough to lead to decreased drug dispersion and limit continued drug administration by this route. (The adhesions that have been found to form during intraperitoneal chemotherapeutic treatment were referred to above as "fibrin-like". These fibrin-like adhesions are composed of fibrin and other substances, including cellular debris, cellular exudates, and other exudates.)

This invention is based on the discovery that when a non-steroidal anti-inflammatory drug ("NSAID") and an antineoplastic chemotherapeutic agent are concurrently administered to the peritoneal cavity, the tendency of the CAA to form adhesions is reduced.

### Brief Summary of the Invention

The invention resides in a combination of a chemotherapeutic antineoplastic agent and a non-steroidal anti-inflammatory drug for simultaneous, separate or sequential use in the treatment of carcinoma in the peritoneal cavity, the non-steroidal anti-inflammatory drug being in an amount sufficient to reduce the tendency of the chemotherapeutic antineoplastic agent to form adhesions.

### Detailed Description of the Invention

The invention is used to alleviate one of the side effects that results from the use of chemotherapeutic antineoplastic agents in the peritoneal cavity. Since the use of CAA's in the peritoneal cavity is known, the factors to be considered in deciding whether or not to carry out such a therapy, which specific CAA to be used in individual cases, and its mode of use (i.e. route and mode of administration, dosage rate, total dosage, time of administration, etc.), are already known in the art, and such knowledge is applicable to this invention. That is, this invention would not change in any material way the manner in which intraperitoneal antineoplastic chemotherapy is carried out. There are many literature references that discuss intraperitoneal chemotherapy. The articles cited above in the Background of the Invention section of this application, and the references cited in these articles, are illustrative and are referred to for illustrative discussions of the use of CAA's in the peritoneal cavity for the treatment of cancer therein.

The chemotherapeutic antineoplastic agents that can be used in the invention, either singly or in combination with one or more other such agents, include, for example, adriamycin, methotrexate, bleomycin, cisplatin, cytarabine, doxorubicin, melphalin, 5-fluorouracil, carboplatin, and mitoxantrone.

The CAA's may be used in combination with antagonists that reduce the toxicity of the CAA's, in accordance with principles that are known in the art. For instance, leucovorin calcium may be administered IV during (or shortly after) intraperitoneal methotrexate therapy to reduce leucopenia, and thiosulfate may be administered IV during intraperitoneal cisplatin therapy to reduce nephrotoxicity (e. g., see the Howell et al. article cited above in the Background of the Invention section of this application).

The NSAID is used with the CAA in an amount sufficient to reduce the tendency of the CAA to form adhesions. The exact quantity and rate of administration of NSAID to be employed in individual cases can be determined by routine experimentation. The examples, below, illustrate some quantities and rates of administration of NSAID (using tolmetin as illustrative) that have been found to be operative in animal model experiments. Similar experiments can be carried out to determine the appropriate quantities and rates of administration of the NSAID in individual cases. It is well within the skill of the art to carry out such experiments.

The NSAID is administered concurrently with the CAA. The terms "concurrent" and "concurrently", with respect to the administration of the CAA and the NSAID, have the following meaning:
The NSAID can be administered in a liquid vehicle that also contains the CAA. The two medicaments can also be administered at the same time as two different compositions from separate reservoirs. The two compositions, one containing the CAA and the other containing the NSAID, may then be introduced into the peritoneal cavity through two separate catheters or the two compositions can be mixed and the resulting mixture can be introduced into the peritoneal cavity through one catheter. Alternatively, the two medicaments can be administered sequentially through the same or different catheters, first one and then the other (which sequence may be repeated a plurality of times). The specific mode of administration is not critical, as long as the NSAID is present in the peritoneal cavity along with the CAA (i. e., is administered "concurrently", as defined herein).

The NSAID may be administered in an isotonic aqueous vehicle such as saline or phosphate buffered saline ("PBS"), and it may be administered as a pharmaceutically acceptable water-soluble salt or ester of the NSAID such as tolmetin sodium. The preparation of aqueous solutions of NSAID's is known in the art. For instance, many NSAID's are soluble in aqueous liquids, and they can be directly dissolved therein simply by mixing. Specific examples of NSAID's that are soluble in isotonic aqueous liquids buffered to a pH between 7 and 8 (the preferred vehicles for administration of the NSAID) include tolmetin sodium, ibuprofen, suprofen, indomethacin sodium trihydrate, sulindac, naproxen sodium, and phenylbutazone. Either the CAA or the NSAID can be administered in an aqueous dispersion or suspension, in addition to an aqueous solution. In some cases, it may be desirable to include a carrier such as hyaluronic acid or a liposome in the aqueous medium. In some cases, either medicament may be employed in the form of a micellar solution or suspension in combination with an aqueous surfactant.

Non-steroidal anti-inflammatory drugs constitute a known class of pharmaceutically active compositions. Specific illustrative examples of NSAID's include ibuprofen, tolmetin, indomethacin, sulindac, suprofen, phenylbutazone, and naproxen, as well as pharmaceutically acceptable salts and esters thereof. The preferred NSAID is tolmetin, which is preferably used in the form of a water-soluble salt such as tolmetin sodium.

The invention is employed in the treatment of cancer in the peritoneal cavity in mammals, including humans. The invention is applicable to the treatment of any kind of cancer in the peritoneal cavity wherein such cancer is treated by the introduction into the peritoneal cavity of a chemotherapeutic antineoplastic agent. Illustrative of such types of cancer are the following:
serous cystadenocarcinoma;
mucinous cystadenocarcinoma;
dysgerminoma;
teratoma;
gastric carcinoma;
pancreatic cancer; and
colonic cancer.

The NSAID is not claimed to be per se a cancer-treating agent. Rather, the NSAID alleviates one of the undesired side effects (i. e., fibrin adhesion formation) that results from the use of CAA's in the peritoneal cavity. Therefore, the invention can be used any time that it is decided to employ a CAA in the peritoneal cavity as an adjunct to the treatment of the cancer by the CAA.

The invention is illustrated in the experimental section below.

Initially, the antineoplastic chemotherapeutic agents adriamycin, bleomycin, and methotrexate, which have been shown to cause adhesion formation in human patients when administered intraperitoneally in the treatment of ovarian cancer, were given to rats via an Alzet osmotic pump (Model 2002, with a delivery rate of 0.5 microliter/hr) placed intraperitoneally. The delivery rate was approximately the same (in terms of mg of drug per kg of body weight) as the dosage rate administered to human patients. When this route of administration did not lead to adhesion formation, and after trying several dosage rates both higher and lower than that given to patients being given such drug therapy, the pumps were placed subcutaneously and the drug was delivered to the peritoneum through a catheter. When vehicle (phosphate buffered saline - "PBS") containing antineoplastic agent was delivered through the catheter, fibrin-like substances formed which covered the catheter in the peritoneum, and adhesions were consistently formed between the catheter, the intestine, and occasionally the liver. Such fibrin-like substances and adhesions did not form when the PBS vehicle control alone was used.

The tables below display the results of tests using chemotherapeutic antineoplastic agents in the rat model described above in which drug delivery continued for the duration of the seven day post-operative period. In each series of tests, a control containing only PBS plus CAA was used, along with several other tests in which tolmetin sodium was also included in the liquid in the pump. The rats were sacrificed seven days post-operatively, and the adhesions were evaluated. The adhesion ratings given have the following significance:
- 0+: No adhesions noted and no covering over the catheter.
- 0.5+: Only a few, very filmy adhesions between the bowel and catheter. Essentially no fibrin-like substance covering the catheter.
- 1.0+: Adhesions present between the lobes of the liver; no adhesions between the intestines. The adhesions between the bowel and the catheter are very filmy but more extensive than in 0.5+. Fibrin-like substance is found covering the catheter.
- 1.5+: Adhesions involve the liver and the bowel as in 2.0+ but the adhesions between the bowel and the catheter are less thick than 2.0+. The covering on the catheter is more evident than 1.0+.
- 2.0+: There are 1-2 points of adherence between the lobes of the liver and a few points of attachment between the loops of bowel. The adhesions between the bowel and the catheter are thick.
- 2.5+: The points of adherence between the lobes of the liver and the bowel are more sparse than in 3+ and there are very thick adhesions between the bowel and the catheter. The catheter is completely covered with fibrin-like substance.
- 3+: Lobes of the liver are joined together; the bowel is adherent to itself and partially adherent to the bladder.
- 4+: Lobes of the liver are joined together; the bowel is adherent to the liver as well as itself. The bowel is stuck to the catheter so that it is difficult to remove.

### Example 1

In the series of experiments in which adriamycin was tested, the concentration of adriamycin in the liquid in the mini-pump was 23.2 µg/ml. The drug was administered to the rat continuously for seven days, after which the animals were sacrificed and rated for extent of adhesions formation. Table I presents the concentration of tolmetin sodium used in each run (the tolmetin sodium was contained in the pump along with the adriamycin), and the adhesion response rating.

**Table I**

| Adriamycin | |
|---|---|
| Tolmetin | Adhesion Rating |
| None | 1.5+, 4+, 3+ |
| 23 mg/ml | 0.5+, 1+, 0 |
| 46 mg/ml | 0.5+, 1+, 0.5+ |

### Example 2

In the next series of experiments bleomycin was evaluated. The liquid in the mini-pump contained either 0.0077 U/ml or 0.77 U/ml of bleomycin, and either none, 23 mg/ml, or 46 mg/ml of sodium tolmetin. (The term "U", used with respect to the dosage of bleomycin, represents "units" of the drug, as defined by the supplier. Those skilled in the art are accustomed to referring to the dosage of bleomycin in this manner.) Table II displays the bleomycin concentrations, the tolmetin sodium concentrations, and the adhesion responses for this series of experiments.

**Table II**

| Bleomycin | | |
|---|---|---|
| Bleomycin | Tolmetin | Adhesion Rating |
| 0.0077 U/ml | None | 2.5+, 4+ |
| 0.77 U/ml | None | 1.5+, 2+, 3.5+ |
| 0.0077 U/ml | 23 mg/ml | 0.5+, 1+, 1.5+ |
| 0.77 U/ml | 23 mg/ml | 0, 0.5+, 0 |
| 0.0077 U/ml | 46 mg/ml | 0.5+, 1+ |
| 0.77 U/ml | 46 mg/ml | 0, 1.5+, 0.5+ |

### Example 3

In this experiment, methotrexate was studied. The methotrexate concentration in the liquid in the mini-pump was 0.77 µg/ml, and the tolmetin sodium concentration was either 0, 23, 46, or 92 mg/ml. Table III displays the concentrations of tolmetin sodium and the adhesion responses found in this experiment.

**Table III**

| Methotrexate | |
|---|---|
| Tolmetin | Adhesion Rating |
| None | 2+, 3+ |
| 23 mg/ml | 1+, 4+ |
| 46 mg/ml | 0.5+, 1.5+ |
| None | 4+, 2.5+, 3.5+ |
| 46 mg/ml | 1.5+, 0.5+, 0 |
| 92 mg/ml | 0, 1+, 0, 1+ |

### Example 4

In this series of experiments mitoxantrone was studied. The concentration of mitoxantrone in the liquid in the mini-pump was 1.16 mg/ml, and the concentration of tolmetin sodium in the liquid in the mini-pump was 0, 23, or 46 mg/ml. Table IV displays the concentrations of tolmetin sodium and the adhesion responses for this series of experiments.

**Table IV**

| Mitoxantrone | |
|---|---|
| Tolmetin | Adhesion Rating |
| None | 3+, 3.5+ |
| 23 mg/ml | 1+, 1+ |
| 46 mg/ml | 0, 1+ |

### Example 5

In the next series of experiments, cisplatin was studied. The concentration of cisplatin in the liquid in the mini-pump was 387 µg/ml, and the concentration of tolmetin sodium in the liquid in the mini-pump was 0, 23, or 46 mg/ml. Table V displays the concentration of tolmetin sodium and the adhesion responses.

**Table V**

| Cisplatin | |
|---|---|
| Tolmetin | Adhesion Rating |
| None | 2+, 4+, 2.5+ |
| 23 mg/ml | 0, 0.5+, 1+ |
| 46 mg/ml | 0.5+, 1+, 1.5+ |

No hard and fast rule can can be set forth about the amount of NSAID that is appropriate to use in individual cases. Experiments such as those set forth above using tolmetin sodium as an exemplary NSAID will have to be carried out for each combination of chemotherapeutic antineoplastic agent and NSAID in order to determine the approximate efficacious dose of NSAID to be used for best results in individual cases. It is well within the skill of the ordinary worker in the art to carry out such experiments in order to determine the appropriate NSAID dosages.

## Claims

1. A combination of a chemotherapeutic antineoplastic agent and a non-steroidal anti-inflammatory drug for simultaneous, separate or sequential use in the treatment of carcinoma in the peritoneal cavity, the non-steroidal anti-inflammatory drug being in an amount sufficient to reduce the tendency of the chemotherapeutic antineoplastic agent to form adhesions, wherein
the chemotherapeutic antineoplastic agent is chosen from the group comprising, either singly or in combination, adriamycin, methotrexate, bleomycin, cisplatin, cytarabine, doxorubicin, melphalin, 5-fluorouracil, carboplatin, and mitoxantrone; and
the non-steroidal anti-inflammatory drug is chosen from the group comprising ibuprofen, tolmetin, indomethacin, sulindac, suprofen, phenylbutazone, naproxen, and pharmaceutically acceptable salts and esters thereof.

2. A combination according to claim 1 wherein the non-steroidal anti-inflammatory drug is tolmetin or a pharmaceutically acceptable salt or ester thereof.

3. A combination according to claim 2 wherein the tolmetin is in the form of the sodium salt.

4. A combination according to any of claims 1 to 3 wherein the chemotherapeutic antineoplastic agent is adriamycin, bleomycin, mitoxantrone, cisplatin, or methotrexate.

5. A combination according to any preceding claim, wherein the chemotherapeutic antineoplastic agent and the non-steroidal anti-inflammatory drug are present as a mixture.

## Patentansprüche

1. Kombination eines chemotherapeutischen antineoplastischen Mittels und eines nicht-steroiden entzündungshemmenden Arzneistoffs zur gleichzeitigen, getrennten oder aufeinanderfolgenden Verwendung bei der Behandlung eines Karzinoms in der Bauchhöhle, wobei der nicht-steroide entzündungshemmende Arzneistoff in einer Menge vorliegt, die ausreicht, die Tendenz des chemotherapeutischen antineoplastischen Mittels, Verklebungen zu bilden, zu vermindern, wobei
das chemotherapeutische antineoplastische Mittel ausgewählt ist aus der Gruppe umfassend, entweder alleine oder in Kombination, Adriamycin, Methotrexat, Bleomycin, Cisplatin, Cytarabin, Doxorubicin, Melphalin, 5-Fluoruracil, Carboplatin und Mitoxantron, und
der nicht-steroide entzündungshemmende Arzneistoff ausgewählt ist aus der Gruppe umfassend Ibuprofen, Tolmetin, Indomethacin, Sulindac, Suprofen, Phenylbutazon, Naproxen und pharmazeutisch verträgliche Salze und Ester davon.

2. Kombination nach Anspruch 1, wobei der nicht-steroide entzündungshemmende Arzneistoff Tolmetin oder ein pharmazeutisch verträgliches Salz oder Ester davon ist.

3. Kombination nach Anspruch 2, wobei das Tolmetin in Form des Natriumsalzes vorliegt.

4. Kombination nach einem der Ansprüche 1 bis 3, wobei das chemotherapeutische antineoplastische Mittel Adriamycin, Bleomycin, Mitoxantron, Cisplatin oder Methotrexat ist.

5. Kombination nach einem der vorhergehenden Ansprüche, wobei das chemotherapeutische antineoplastische Mittel und der nicht-steroide entzündungshemmende Arzneistoff als ein Gemisch vorliegen.

## Revendications

1. Combinaison d'un agent chimiothérapeutique antinéoplasique et d'un médicament anti-inflammatoire non stéroïdien pour l'utilisation simultanée, séparée ou successive dans le traitement d'un cancer de la cavité péritonéale, le médicament anti-inflammatoire non stéroïdien étant en une quantité suffisante pour réduire la tendance de l'agent chimiothérapeutique antinéoplasique à former des adhérences, dans laquelle
l'agent chimiothérapeutique antinéoplasique est choisi dans le groupe constitué, séparément ou en combinaison, de l'adriamycine, du méthotrexate, de la bléomycine, du cisplatine, de la cytarabine, de la doxorubicine, du melphalan, du 5-fluoro-uracile, du carboplatine et de la mitoxantrone ; et
le médicament anti-inflammatoire non stéroïdien est choisi dans le groupe comprenant l'ibuprofène, la tolmétine, l'indométhacine, le sulindac, le suprofène, la phénylbutazone, le naproxen et leurs sels et esters pharmaceutiquement acceptables.

2. Combinaison selon la revendication 1, dans laquelle le médicament anti-inflammatoire non stéroïdien est la tolmétine ou un sel ou ester pharmaceutiquement acceptables de celle-ci.

3. Combinaison selon la revendication 2, dans laquelle la tolmétine est sous forme du sel de sodium.

4. Combinaison selon l'une quelconque des revendications 1 à 3, dans laquelle l'agent chimiothérapeutique antinéoplasique est l'adriamycine, la bléomycine, la mitoxantrone, le cisplatine ou le méthotrexate.

5. Combinaison selon l'une quelconque des revendications précédentes, dans laquelle l'agent chimiothérapeutique antinéoplasique et le médicament anti-inflammatoire non stéroïdien sont présents sous forme d'un mélange.
